# EUROPÄISCHE PATENTANMELDUNG

(11) **EP 1 469 006 A2**
(43) Veröffentlichungstag der Anmeldung: **20.10.2004**
(21) Anmeldenummer: 04001905.1
(22) Anmeldetag: 29.01.2004
(51) Int. Cl.: C07F 15/00, B01J 31/18, C07B 53/00

(54) **Verfahren zur Reduktion von Ketocarbonsäureestern**

(30) Priorität: 12.02.2003 DE 10305946
(71) Anmelder: Bayer Chemicals AG, 51368 Leverkusen (DE)
(72) Erfinder: Thomas, John Meurig, Prof. Dr. Sir, Cambridge CB2 2PN (GB); Johnson, Brian F. G., Prof. Dr., Cambridge CB3 0DG (GB); Raja, Robert, Dr., Cambridge CB4 6DG (GB); Jones, Matthew David, Cambridge DB3 0HF (GB)

(57) **Zusammenfassung**

Die vorliegende Erfindung betrifft ein Verfahren zur Herstellung von enantiomerenangereicherten α- und β-Hydroxycarbonsäureestern aus den entsprechenden Ketocarbonsäureestem sowie dafür verwendbare immobilisierte Übergangsmetallkomplexe.

## Beschreibung

Die vorliegende Erfindung betrifft ein Verfahren zur Herstellung von enantiomerenangereicherten α- und β-Hydroxycarbonsäureestem aus den entsprechenden Ketocarbonsäureestem sowie dafür verwendbare immobilisierte Übergangsmetallkomplexe.

Enantiomerenangereicherte α- und β-Hydroxycarbonsäureester sind wertvolle Reagenzien zur Racematspaltung und wichtige Intermediate bei der Herstellung von Arzneimitteln und Agrochemikalien. Üblicherweise werden enantiomerenangereicherte α- und β-Hydroxycarbonsäureester durch katalytische Hydrierung der entsprechenden α- und β-Ketocarbonsäureester gewonnen, wobei als Katalysatoren häufig Übergangsmetallkomplexe mit chiralen Phosphanen als Liganden eingesetzt werden (siehe z.B. Genet et al., Tetrahedron, Asymmetry, 1994, 5(4), 675-690). Nachteilig an chiralen Phosphanen ist der hoher Preis und die Oxidationsempfindlichkeit weshalb ihr Einsatz im industriellen Maßstab wenn überhaupt überwiegend in homogenen Prozessen erfolgt.

Alternativ dazu sind Verfahren unter Verwendung von mit Chinchona-Alkaloiden oder Weinsäurederivaten modifizierten Platin- oder Nickelkatalysatoren bekannt (T. Mallat et al., Fine Chemicals through Heterogeneous Catalysis, Wiley-VCH, 2001, S. 449 ff).

Darüber hinaus wird für die Hydrierung von Ketoestern in Ferrand et al. (Tetrahedron: Asymmetry, 13, 2002, S. 1379 bis 1384) der Einsatz von Rhodium-, Ruthenium- und Iridium-Komplexen mit chiralen Diaminen beschrieben. Allen Verfahren ist jedoch gemeinsam, dass sie allenfalls einen mäßigen Enantiomerenüberschuss erlauben.

Es bestand daher das Bedürfnis, Katalysatoren bereitzustellen, die insbesondere in einem Verfahren zur Herstellung von enantiomerenangereicherten α- und β-Hydroxycarbonsäureestern hohe Ausbeuten und Enantioselektivitäten ermöglichen.

Es wurden nun Verbindungen der Formel (I) gefunden, 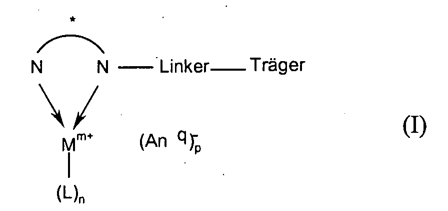 in der 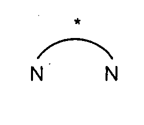 für eine enantiomerenangereicherte chirale Stickstoffverbindung steht,
- Linker: für einen Rest steht, der sowohl kovalent an die enantiomerenangereicherte chirale Stickstoffverbindung als auch an den Träger gebunden ist,
- Träger: für ein mikro-, meso- oder makroporöses Trägermaterial steht,
- (M ^{m+}): für ein Metall mit der Wertigkeit m steht
- L: für einen anionischen oder neutralen Liganden steht
- n: für eins, zwei, drei oder vier steht
- (An ^{q-}): für ein Anion mit der Wertigkeit q steht und
- p: (m - Anzahl der anionischen Liganden L)/q ist.

Enantiomerenangereicherte Verbindungen im Sinne der Erfindung sind enantiomerenreine Verbindungen oder Mischungen von Enantiomeren einer Verbindung, in denen ein Enantiomer in einem Enantiomerenüberschuss, im Folgenden auch ee (enantiomeric excess) genannt, im Vergleich zum anderen Enantiomer vorliegt. Bevorzugt beträgt dieser Enantiomerenüberschuss 10 bis 100 % ee, besonders bevorzugt 90 bis 100 % ee und ganz besonders bevorzugt 95 bis 100 % ee.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Im Folgenden stehen **Alkyl** beziehungsweise **Alkoxy** beziehungsweise **Alkylen** beziehungsweise **Alkenylen** jeweils unabhängig für einen geradkettigen, cyclischen, verzweigten oder unverzweigten Alkyl- beziehungsweise Alkoxy- beziehungsweise Alkylen- beziehungsweise Alkenylen-Rest, der gegebenenfalls weiter durch C₁-C₄-Alkoxy substituiert sein kann. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₂₀-Alkyl weiter darüber hinaus beispielsweise für Adamantyl, die isomeren Menthyle, n-Nonyl, n-Decyl und n-Dodecyl.

C₁-C₄-Alkoxy steht beispielsweise für Methoxy, Ethoxy, n-Propoxy, iso-Propoxy, n-Butoxy, sec.-Butoxy und tert.-Butoxy, C₁-C₈-Alkoxy darüberhinaus beispielsweise für n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy, C₁-C₂₀-Alkoxy weiter darüber hinaus beispielsweise für Adamantoxy, die isomeren Menthoxy-Reste, n-Decoxy und n-Dodecoxy.

C₁-C₄-Alkylen steht beispielsweise für Methylen, 1,1-Ethylen, 1,2-Ethylen, 1,1-Propylen, 1,3-Propylen, 1,4-Butylen, C₁-C₈-Alkylen darüberhinaus beispielsweise für 1,2-cyclo-Hexylen und 1,2-cyclo-Pentylen.

C₂-C₈-Alkenylen steht beispielsweise für 1,1-Ethenylen, 2-Ethoxy-1,1-ethenylen und 2-Methoxy-1,1-ethenylen.

**Halogenalkyl,** beziehungsweise **Halogenalkoxy,** beziehungsweise **Halogenalkylen** bedeutet jeweils unabhängig einen geradkettigen, cyclischen, verzweigten oder unverzweigten Alkyl-Rest, beziehungsweise Alkoxy-Rest, beziehungsweise Alkylen-Rest, der einfach, mehrfach oder vollständig durch Halogenatome substituiert ist.

Beispielsweise steht C₁-C₂₀-Halogenalkyl für Trifluormethyl, Chlormethyl, 2-Chlorethyl, 2,2,2-Trifluorethyl, Pentafluorethyl, Nonafluorbutyl, Heptafluorisopropyl, Perfluoroctyl, Perfluordodecyl und Perfluorhexadecyl.

**Aryl** steht jeweils unabhängig für einen heteroaromatischen Rest mit 5 bis 14 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können und vorzugsweise für einen carbocyclischen aromatischen Rest mit 6 bis 14 Gerüstkohlenstoffatomen.

Beispiele für carbocyclische aromatische Reste mit 6 bis 14 Gerüstkohlenstoffatomen sind Phenyl, Biphenyl, Naphtyl, Phenanthrenyl, Anthracenyl oder Fluorenyl, heteroaromatische Reste mit 5 bis 14 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridinyl, Oxazolyl, Benzofuranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die beispielsweise ausgewählt sind aus der Gruppe Nitro, Cyano, Chlor, Fluor, C₁-C₁₂-Alkyl, C₁-C₁₂-Halogenalkyl, C₁-C₁₂-Halogenalkoxy, C₁-C₁₂-Halogenalkylthio, C₁-C₁₂-Alkoxy, Di(C₁-C₈-alkyl)amino oder Tri(C₁-C₆-alkyl)siloxyl.

**Arylen** steht für einen Aryl-Rest, der eine weitere Bindungsstelle am aromatischen Gerüst besitzt und dadurch divalent ist.

**Arylalkyl** bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann.

**Arylalkylen** steht für einen Arylalkyl-Rest, der eine weitere Bindungsstelle am aromatischen Gerüst besitzt und dadurch divalent ist.

Im Folgenden werden Vorzugsbereiche für Verbindungen der Formel (I) definiert: 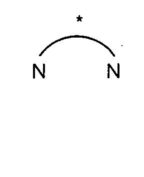 steht bevorzugt für eine enantiomerenangereicherte chirale Stickstoffverbindung der Formel (II) 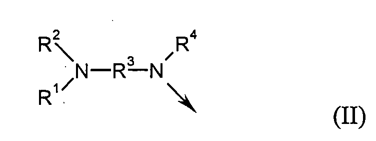 in der
der Pfeil die Bindungsstelle zum Linker anzeigt und
- R¹, R² und R⁴: jeweils unabhängig voneinander für Wasserstoff, C₁-C₈-Alkyl, C₅-C₁₅-Arylalkyl oder C₄-C₁₄-Aryl stehen oder NR¹R² als Ganzes für einen cyclischen Aminorest mit insgesamt 4 bis 20 Kohlenstoffatomen steht,
- R³: für einen divalenten Rest mit 2 bis 30 Kohlenstoffatomen steht oder
- R³: und mindestens einer der Reste R¹, R² und R⁴ zusammen Teil eines cyclischen Aminorestes mit insgesamt 4 bis 20 Kohlenstoffatomen sind.

Bevorzugte Verbindungen der Formel (II) sind solche, in der

R¹, R² und R⁴ jeweils unabhängig voneinander für Wasserstoff, C₁-C₈-Alkyl, C₅-C₁₅-Arylalkyl oder C₄-C₁₄-Aryl stehen oder NR¹R² als Ganzes für einen 5- oder 6-gliedrigen monocyclischen Aminorest steht, der am Kohlenstoffgerüst gegebenenfalls einfach, zweifach, dreifach oder vierfach C₁-C₄-Alkyl substituiert ist und
R³ für einen divalenten Rest steht, der ausgewählt ist aus der Gruppe C₂-C₈-Alkylen, das gegebenenfalls einfach oder zweifach durch C₄-C₁₄-Arylreste weiter substituiert sein kann, C₅-C₁₅-Arylalkylen, C₄-C₁₄-Arylen oder Bis-(C₄-C₁₄-arylen) oder
R³ und einer der Reste R¹, R² und R⁴ zusammen Teil eines 5- oder 6-gliedrigen monocyclischen Aminorestes sind, der am Kohlenstoffgerüst gegebenenfalls zusätzlich einfach, zweifach, dreifach oder vierfach durch C₁-C₄-alkyl substituiert ist.

Besonders bevorzugte Verbindungen der Formel (II) sind solche, in denen
R¹, R² und R⁴ jeweils unabhängig voneinander für Wasserstoff, Methyl oder Ethyl stehen und
R³ für einen divalenten Rest steht, der ausgewählt ist aus der Gruppe 1,2-Bis-(C₄-C₁₄-aryl)-1,2-ethylen, 1,2-Cyclohexylen, 1,1'-2,2'-Bis-(C₄-C₁₄-arylen) oder

R³ und einer der Reste R¹, R² und R⁴ zusammen Teil eines Pyrrolidinyl- oder Piperidinyl-Restes sind.

Ganz besonders bevorzugte Verbindungen der Formel (II) sind solche, die sich von folgenden Verbindungen ableiten:
(1R,2R)-1,2-Diphenylethylendiamin, (1S,2S)-1,2-Diphenylethylendiamin, (1R,2R)-1,2-Dimethylethylendiamin, (1S,2S)-1,2-Dimethylethylendiamin, (1R,2R)-1,2-cyclohexylendiamin, (1S,2S)-1,2-cyclohexylendiamin, (S)-2-Aminomethyl-1-ethylpyrrolidin, (R)-2-Aminomethyl-1-ethylpyrrolidin, (S)-2-Aminomethyl-1-methylpyrrolidin, (R)-2-Aminomethyl-1-methylpyrrolidin, (R)-1,1'-Diamino-2,2'binaphthyl, (S)-1,1'-Diamino-2,2'-binaphthyl, (R)-1,1'-Diamino-6,6'-Dimethoxy-2,2'-biphenyl und (S)-1,1'-Diamino-6,6'-Dimethoxy-2,2'-biphenyl, wobei (R)-2-Aminomethyl-1-ethylpyrrolidin und (S)-2-Aminomethyl-1-methylpyrrolidin noch weiter bevorzugt sind.

**Träger** steht bevorzugt für ein mikro- oder mesoporöses Trägermaterial. Die im Rahmen der Erfindung geltenden Definitionen für die Begriffe mikro-, meso- und makroporös wie auch die Nomenklatur der Zeolithe sind dabei IUPAC-konform auszulegen (McCusker et al. Pure Appl. Chem, vol. 73, No. 2, pp 381-394, 2001). Geeignete Trägermaterialien sind beispielsweise Silica-Gele, Zeolithe vom Typ MOR, X, Y, MCM, ZSM, FAU, MFI, L, BEA, FER, A und SBA, sowie solche vom Typ AlPO, MAlPO und SAPO, wobei die genannten Zeolithe gegebenenfalls isomorph substituiert sein können. Besonders bevorzugt sind mesoporöse Zeolithe wie insbesondere solche des MCM-Typs wie beispielsweise MCM-41.

Bevorzugte Kombinationen **Linker-Träger** sind solche, die dadurch erhältlich sind, dass zunächst ein Träger an der Oberfläche in der Weise organisch modifiziert wird, dass er nach der Modifizierung eine oder mehrere Funktionalitäten aufweist (im Folgenden **aktivierter Träger** genannt) und über diese Funktionalitäten die Verknüpfung mit den vorstehend definierten enantiomerenangereicherten chiralen Stickstoffverbindungen erfolgt. Besonders bevorzugte Funktionalitäten sind dabei solche, die mit Aminen unter Erhöhung der Wertigkeit des Amins reagieren können (z.B. mit sekundären Aminen tertiäre Amine bilden oder mit primären Aminen sekundäre Amine bilden). Solche Funktionalitäten sind beispielsweise Chlor-, Bromoder Iodatome, sowie Perfluoracylat- oder Sulfonat-Reste.

Die beschriebenen organischen Modifizierungen, d.h. die Herstellungsmethoden für aktivierte Träger sind hinreichend bekannt und können in an sich bekannter Weise beispielsweise durchgeführt werden durch
- Umsetzung des Trägers mit einem Chlorierungsmittel wie z.B. Tetrachlorkohlenstoff, Thionylchlorid, Titantetrachlorid oder Phosphorpentachlorid (siehe auch Beck et al. J. Am. Chem. Soc 1992, 114, 10834) und anschließende Umsetzung mit funktionalisierten Alkoholen oder Alkoxysilanen oder
- Umsetzung des Trägers mit Siliziumtetrachlorid, anschließende Umsetzung mit sekundären Aminen und weitere Umsetzung mit funktionalisierten Alkoholen oder Alkoxysilanen (siehe auch Petrucci et al. Bull. Chem. Soc. Japan, 1990, 63, 988) Silazanen oder vorzugsweise durch
- Umsetzung des Trägers mit Siliziumtetrachlorid oder Chlorsilanen des Typs SiClᵣ(C₁-C₈-Alkyl)ₛ(C₄-C₁₄-Aryl)ₜ(C₅-C₁₅-Arylalkyl)ᵤ, wobei r für eins, zwei oder drei steht und r+s+t+u = 4 ist und anschließende Umsetzung mit funktionalisierten Alkoholen, Chlorsilanen oder Alkoxysilanen.

Besonders bevorzugte **aktivierte Träger** sind solche, die durch Umsetzung von Trägem mit Chlorsilanen des Typs SiClᵣ(C₁-C₈-Alkyl)ₛ(C₄-C₁₄-Aryl)ₜ(C₅-C₁₅-Aryl-alkyl)ᵤ, wobei r für eins, zwei oder drei steht und r+s+t+u = 4 ist und anschließender Umsetzung mit funktionalisierten Alkoxysilanen oder Halogensilanen der Formeln (IIIa) oder (IIIb) erhältlich sind,

Hal-(C₂-C₁₂-Alkylen)-Si[O(C₁ -C₈-Alkyl)]₃ (IIIa)

Hal-(C₂-C₁₂-Alkylen)-SiHal₃ (IIIb)

wobei in Formel (III)
- Hal: für Chlor oder Brom steht.

Ganz besonders bevorzugte **aktivierte Träger** sind solche, die erhalten werden durch Umsetzung von Trägem mit Diphenyldichlorsilan und anschließende Umsetzung mit 3-Brompropyltrichlorsilan.

In Formel (I) steht weiterhin
- (M ^{m+}): vorzugsweise für Cobalt in den formalen Oxidationsstufen 0, +2 und +3, Rhodium und Iridium in den formalen Oxidationsstufen +1 und +3, Nickel, Palladium und Platin in den formalen Oxidationsstufen 0 und +2 sowie Ruthenium in der formalen Oxidationsstufe +2, wobei Rh^{I}, Ir^{I} und Pd^{II} bevorzugt sind.
- L: steht bevorzugt für folgende Ligandentypen: Monoolefine wie beispielsweise Ethylen, Cycloocten und Cyclohexen, Diolefine wie beispielsweise 1,5-Cyclooctadien (cod), Norbornadien (nbd), und Butadien, Nitrile wie Acetonitril (ACN), Benzonitril und Benzylnitril, Aromaten wie Benzol, Mesitylen und Cymol, sowie anionische Liganden wie Allyl, Methylallyl, Phenylallyl, C₁-C₈-Alkylacylacetonate, C₁-C₈-Alkylacylate, Chlorid, Bromid und Iodid.
- (An ^{q-}): steht bevorzugt für nicht oder schwach koordinierende Anionen wie beispielsweise Nitrat, Perchlorat, Sulfat, Hexafluorophosphat, Hexafluoroantimonat, Hexachloroantimonat, Borate wie beispielsweise Tetrafluoroborat und Tetraphenylborat oder Sulfonate wie beispielsweise Trifluormethansulfonat und Nonafluorbutansulfonat.

Besonders bevorzugt steht 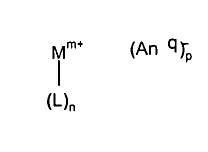 als ganzes Fragment für Rh(cod)BF₄, Ir(cod)BF₄, Rh(cod)PF₆, Ir(cod)PF₆, Rh(cod)SbF₆, Ir(cod)SbF₆, Rh(cod)ClO₄, Ir(cod)ClO₄, Rh(nbd)BF₄, Ir(nbd)BF₄, Rh(nbd)PF₆, Ir(nbd)PF₆, Rh(nbd)SbF₆, Ir(nbd)SbF₆, Rh(nbd)ClO₄, Ir(nbd)ClO₄, Pd(Allyl)BF₄, Pd(Allyl)PF₆ und Pd(ACN)₂(BF₄)₂.

Ganz besonders bevorzugte Verbindungen der Formel (I) sind solche der Formeln (Ia), (Ib), (Ic) und (Id) 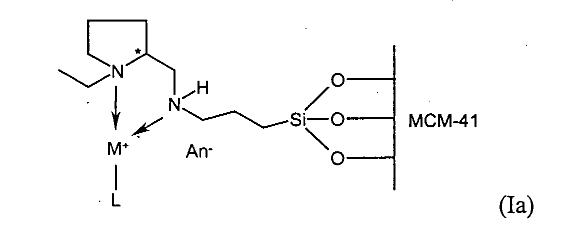 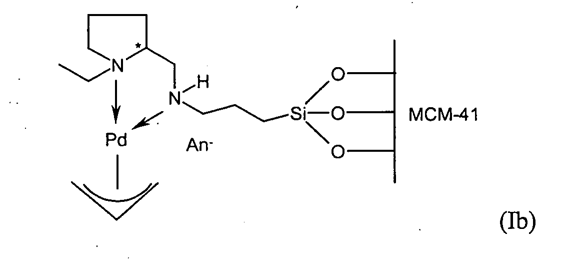 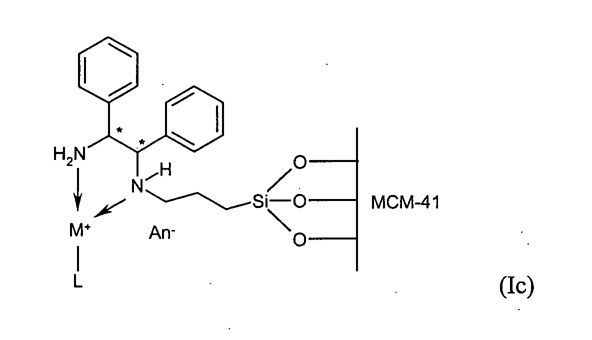 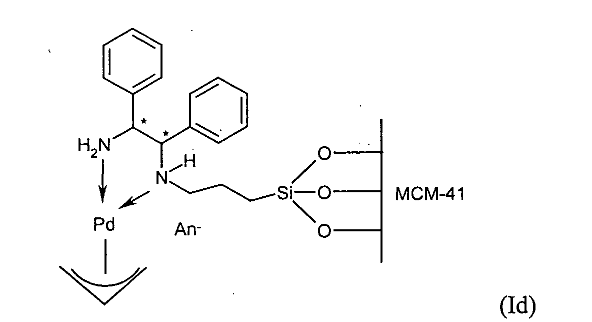 in denen jeweils
- *: ein stereogenes Zentrum markiert, das entweder R oder S konfiguriert ist, wobei die Auflage gilt, dass Mesoformen ausgeschlossen sind (Verbindungen der Formel (Ic) und (Id))
- M⁺: für Rhodium^{I} oder Iridium^{I} steht und
- L: für cod oder nbd steht und
- An⁻: für Perchlorat, Hexafluorophosphat, Trifluormethansulfonat oder Tetrafluoroborat steht.

Zur Herstellung der Verbindungen der Formel (I) wie insbesondere solchen der Formeln (Ia) bis (Id) geht man vorzugsweise so vor, dass
in einem Schritt A) ein aktivierter Träger der Formel (IV)

Hal-Linker-Träger (IV)

in der Linker und Träger die unter der Formel (I) genannten Bedeutungen und Vorzugsbereiche besitzen
mit enantiomerenangereicherten chiralen Stickstoffverbindurigen der Formel (IIa) 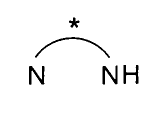 für die die unter der Formel (I) genannten Bedeutungen und Vorzugsbereiche gelten gegebenenfalls in Gegenwart eines organischen Lösungsmittels umgesetzt wird und in einem Schritt B) die auf diese Weise erhaltenen Verbindungen der Formel (V) 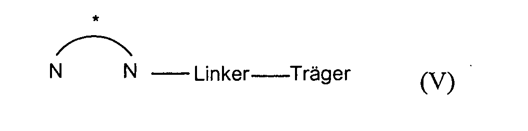 gegebenenfalls in Gegenwart eines organischen Lösungsmittels mit Übergangsmetallverbindungen zu Verbindungen der Formel (I) umgesetzt werden.

Die Verbindungen der Formel (V) sind von der Erfindung ebenfalls umfasst, wobei die unter der Formel (I) angegebenen Vorzugsbereiche in gleicher Weise gelten.

Bevorzugte Übergangsmetallverbindungen sind solche der Formel (VIa)

M¹(An¹)ₚ₁ (VIa)

in der
- M¹: für Ruthenium, Rhodium, Iridium, Nickel, Palladium oder Platin und
- An¹: für Chlorid, Bromid, Acetat, Nitrat, Methansulfonat, Trifluormethansulfonat oder Acetylacetonat und
- p¹: für Ruthenium, Rhodium und Iridium für 3, für Nickel, Palladium und Platin für 2 steht,
oder Übergangsmetallverbindungen der Formel (VIb)

M²(An²)ₚ₂L¹ ₂ (VIb)

in der
- M²: für Ruthenium, Rhodium, Iridium, Nickel, Palladium oder Platin und
- An²: für Chlorid, Bromid, Acetat, Methansulfonat, Trifluormethansulfonat, Tetrafluoroborat, Hexafluorophosphat, Perchlorat, Hexafluoroantimonat, Tetra(bis-3,5-trifluormethylphenyl)borat oder Tetraphenylborat steht und
- p2: für Rhodium und Iridium für 1, für Nickel, Palladium, Platin und Ruthenium für 2 steht und
- L¹: jeweils für ein C₂-C₁₂-Alken wie beispielsweise Ethylen oder Cycloocten, oder ein Nitril wie beispielsweise Acetonitril, Benzonitril oder Benzylnitril steht, oder
- L¹ ₂: zusammen für ein (C₄-C₁₂)-Dien wie beispielsweise Norbornadien oder 1,5-Cyclooctadien steht
oder Übergangsmetallverbindungen der Formel (VIc)

[M³L²An³ ₂]₂ (VIc)

in der
- M³: für Ruthenium und
- L²: für cod, nbd, Allyl, Methylallyl oder Arylreste wie zum Beispiel Cymol, Mesitylen, Benzol und
- An³: für Chlorid, Bromid, Acetat, Methansulfonat, Trifluormethansulfonat, Tetrafluoroborat, Hexafluorophosphat, Perchlorat, Hexafluoroantimonat, Tetra(bis-3,5-trifluormethylphenyl)borat oder Tetraphenylborat steht
oder Übergangsmetallverbindungen der Formel (VId)

M⁴ ₚ₃ [M⁵(An³)₄] (VId),

wobei
- M⁵: für Palladium, Nickel, Iridium oder Rhodium und
- An³: für Chlorid oder Bromid steht und
- M⁴: für Lithium, Natrium, Kalium, Ammonium oder organisches Ammonium steht und
- p³: für Rhodium und Iridium für 3, für Nickel, Palladium und Platin für 2 steht,
oder Übergangsmetallverbindungen der Formel (VIe)

[M⁶(L³)₂]An⁴ (VIe),

wobei
- M⁶: für Iridium oder Rhodium und
- L³: für ein (C₄-C₁₂)-Dien wie beispielsweise Norbomadien oder 1,5-Cyclooctadien steht und
- An⁴: für ein nicht oder schwach koordinierendes Anion wie zum Beispiel Methansulfonat, Trifluormethansulfonat, Tetrafluoroborat, Hexafluoro-phosphat, Perchlorat, Hexafluoroantimonat, Tetra(bis-3,5-trifluormethylphenyl)borat oder Tetraphenylborat steht.

Darüber hinaus sind als Übergangsmetallverbindungen beispielsweise Ni(cod)₂, Pd₂(dibenzylidenaceton)₃, Cyclopentadienyl₂Ru, Rh(acetylacetonat)(CO)₂, Ir(pyridin)₂(cod)OTf oder mehrkernige verbrückte Komplexe wie beispielsweise [Pd(Allyl)Cl]₂, [Pd(Allyl)Br]₂, [Rh(cod)Cl]₂, [Rh(cod)Br]₂, [Rh(Ethen)₂Cl]₂, [Rh(Cycloocten)₂Cl]₂, [Ir(cod)Cl]₂ und [Ir(cod)Br]₂, [Ir(Ethen)₂Cl]₂ und [Ir(Cycloocten)₂Cl]₂ geeignet.

Besonders bevorzugte Übergangsmetallverbindungen sind: [Pd(Allyl)Cl]₂, [Pd(Allyl)Br]₂, [Rh(cod)Cl]₂, [Rh(cod)₂Br], [Rh(cod)₂]ClO₄, [Rh(cod)₂]BF₄, [Rh(cod)₂]PF₆, [Rh(cod)₂]Otf (Otf = Triflat), [Rh(cod)₂]BPh₄, [Rh(cod)₂]SbF₆ RuCl₂(cod), [(Cymol)RuCl₂]₂, [(Benzol)RuCl₂]₂, [(Mesitylen)RuCl₂]₂, [(Cymol)RuBr₂]₂, [(Cymol)RuI₂]₂, [(Cymol)Ru(BF₄)₂]₂, [(Cymol)Ru(PF₆)₂]₂, [(Cymol)Ru(BPh₄)₂]₂, [(Cymol)Ru(SbF₆)₂]₂, [Ir(cod)₂Cl]₂, [Ir(cod)₂]PF₆, [Ir(cod)₂]ClO₄, [Ir(cod)₂]SbF₆ [Ir(cod)₂]BF₄, [Ir(cod)₂]OTf, [Ir(cod)₂]BPh₄, [Rh(nbd)Cl]₂ (nbd = Norbomadien), [Rh(nbd)₂Br], [Rh(nbd)₂]ClO₄, [Rh(nbd)₂]BF₄, [Rh(nbd)₂]PF₆, [Rh(nbd)₂]OTf, [Rh(nbd)₂]BPh₄, [Rh(nbd)₂]SbF₆ RuCl₂(nbd), [Ir(nbd)₂]PF₆, [Ir(nbd)₂]ClO₄, [Ir(nbd)₂]SbF₆ [Ir(nbd)₂]BF₄, [Ir(nbd)₂]OTf, [Ir(nbd)₂]BPh₄, Ir(pyridin)₂(nbd)OTf, [Ru(DMSO)₄Cl₂], [Ru(ACN)₄Cl₂], [Ru(PhCN)₄Cl₂] und [Ru(cod)Cl₂]ₙ, wobei [Pd(Allyl)Cl]₂, Rh(cod)₂OTf, Rh(cod)₂PF₆, Rh(cod)₂SbF₆, Ir(cod)₂BF₄, Rh(cod)₂OTf, Ir(cod)₂PF₆, Ir(cod)₂SbF₆ und Ir(cod)₂BF₄ noch weiter bevorzugt sind.

Es sei darauf hingewiesen, dass es bei Verwendung von halogenidhaltigen Übergangsmetallverbindungen oftmals vorteilhaft ist, zusätzlich in etwa äquimolarer Menge zum vorhandenen Halogenid Silber- oder Kalium-Salze von nicht oder schwach koordinierenden Anionen gemäß oben stehender Definition einzusetzen.

Als organische Lösungsmittel für die Schritte A) und B) eignen sich üblicherweise aliphatische oder aromatische, gegebenenfalls halogenierte Kohlenwasserstoffe, wie beispielsweise Petrolether, Benzol, Toluol, die isomeren Xylole, Chlorbenzol, die isomeren Dichlorbenzole, Hexan, Cyclohexan, Dichlormethan oder Chloroform sowie vorzugsweise Ether, wie Diethylether, Diisopropylether, Dioxan, Tetrahydrofuran, Methyl-tert.-butylether oder Ethylenglykoldimethyl- oder -diethylether. Besonders bevorzugte organische Lösungsmittel sind Toluol, Diethylether, Tetrahydrofuran und Methyl-tert.-butylether.

Das Gewichtsverhältnis von enantiomerenangereichterten chiralen Stickstoffverbindungen zu aktiviertem Träger kann beispielsweise und bevorzugt 0,02:1 bis 100:1, besonders bevorzugt 0,1:1 bis 5:1 und ganz besonders bevorzugt 0,1:1 bis 1:1 betragen. Der Einsatz größerer Mengen von enantiomerenangereichterten chiralen Stickstoffverbindungen ist möglich aber unwirtschaftlich.

Das Gewichtsverhältnis von Übergangsmetallverbindung zu Verbindungen der Formel (V) kann beispielsweise und bevorzugt 0,005:1 bis 1:1, besonders bevorzugt 0,01:1 bis 0,2:1 und ganz besonders bevorzugt 0,02:1 bis 0,1:1 betragen.

Im Schritt A) beträgt die Reaktionstemperatur beispielsweise und bevorzugt 0 bis 100°C, besonders bevorzugt 20 bis 80°C und ganz besonders bevorzugt 40 bis 60°C.

Im Schritt B) beträgt die Reaktionstemperatur beispielsweise und bevorzugt -20°C bis 80°C, besonders bevorzugt 0 bis 60°C und ganz besonders bevorzugt 10 bis 30°C.

Die Aufarbeitung von Verbindungen der Formel (I) kann in an sich bekannter Weise durch Filtration und/oder Zentrifugation und/oder Sedimentation und gegebenenfalls anschließendem Waschen mit organischem Lösungsmittel erfolgen, wobei das Waschen beispielsweise diskontinuierlich oder kontinuierlich durchgeführt werden kann. Zu Lagerzwecken werden die Verbindungen der Formel (I) vorzugsweise getrocknet.

Die Verbindungen der Formel (I) können direkt als Katalysator für asymmetrische Reaktionen eingesetzt werden.

Von der Erfindung sind daher auch Katalysatoren umfasst, die Verbindungen der Formel (I) enthalten.

Weiterhin ist von der Erfindung ein Verfahren zur katalytischen Herstellung von enantiomerenangereicherten Verbindungen umfasst, das dadurch gekennzeichnet ist, dass als Katalysatoren solche eingesetzt werden, die Verbindungen der Formel (I) enthalten.

Bevorzugte Verfahren zur Herstellung von enantiomerenangereicherten Verbindungen sind asymmetrische Hydrierungen, wie beispielsweise Hydrierungen von prochiralen C=C-Bindungen wie prochiralen Enaminen, Olefinen, Enolethern; C=O-Bindungen wie prochiralen Ketonen und C=N-Bindungen wie prochiralen Iminen. Besonders bevorzugte asymmetrische Hydrierungen sind Hydrierungen von prochiralen Ketonen wie insbesondere α- und β-Ketocarbonsäureestern.

Bevorzugte α- und β-Ketocarbonsäureester sind Verbindungen der Formel (VII), 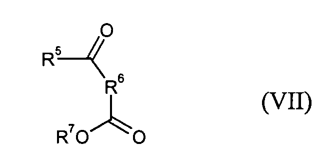 in der
- R⁵ und R⁷: jeweils unabhängig voneinander für C₁-C₁₂-Alkyl, C₁-C₁₂-Halogenalkyl, C₅-C₁₅-Arylalkyl oder C₄-C₁₄-Aryl stehen und
- R⁶: fehlt oder für 1,1-(C₁-C₄-Alkylen) steht.

Vorzugsweise stehen
R⁵ und R⁷ jeweils unabhängig voneinander für C₁-C₄-Alkyl oder Phenyl und R⁶ für Methylen oder fehlt.

Besonders bevorzugte Verbindungen der Formel (VII) sind Phenylglyoxylsäuremethylester und Chloracetessigester.

Durch erfindungsgemäße Hydrierung von α- und β-Ketocarbonsäureestern sind enantiomerenangereicherte Verbindungen der Formel (VIII) erhältlich, 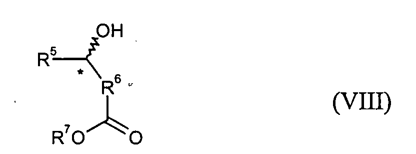 in der
- *: ein stereogenes Zentrum markiert, das S oder R-konfiguriert ist und
- R⁵, R⁶ und R⁷: die unter der Formel (VII) angegebenen Bedeutungen und Vorzugsbereiche besitzen.

Die erfindungsgemäß herstellbaren Verbindungen eignen sich insbesondere als Racematspaltungsreagenzien oder in einem Verfahren zur Herstellung von Arzneimitteln oder Agrochemikalien.

In einer bevorzugten Ausführungsform von erfindungsgemäßen asymmetrischen Hydrierungen beträgt die Reaktionstemperatur 0 bis 200°C, bevorzugt 10 bis 150°C, der Wasserstoffpartialdruck beispielsweise 0,1 bis 200 bar, bevorzugt 0,9 bis 100 bar und besonders bevorzugt 4 bis 30 bar.

Als Lösungsmittel für erfindungsgemäße asymmetrische Hydrierungen eignen sich insbesondere aliphatische oder aromatische, gegebenenfalls halogenierte Kohlenwasserstoffe, wie beispielsweise Petrolether, Benzol, Toluol, die isomeren Xylole, Chlorbenzol, die isomeren Dichlorbenzole, Hexan, Cyclohexan, Dichlormethan oder Chloroform, Ether, wie Diethylether, Diisopropylether, Dioxan, Tetrahydrofuran, Methyl-tert.-butylether oder Ethylenglykoldimethyl- oder -diethylether sowie vorzugsweise Alkohole wie Methanol, Ethanol und Iso-Propanol.

Das Gewichtsverhältnis von Verbindungen der Formel (I) zu Substrat kann beispielsweise 1:1 bis 1:10000 betragen, bevorzugt ist 1:5 bis 1:1000.

Der Vorteil der vorliegenden Erfindung ist, dass auf effiziente Weise heterogene Katalysatoren in hohen Ausbeuten hergestellt werden können und diese Katalysatoren hohe Umsätze und Enantioselektivitäten in asymmetrischen Synthesen erlauben. Dieser Umstand ist insofern als besonders überraschend einzustufen, dass die nicht immobilisierten Analoga zu Verbindungen der Formel (I) wenn überhaupt nur sehr geringe Enantioselektivitäten erlauben, wie die Vergleichsbeispiele zeigen.

### Beispiele

### Beispiel 1 (zum Vergleich)

### Herstellung von [Palladium(η³-Allyl)((S)-2-Aminomethyl-1-ethylpyrrolidin)]BF₄

[Pd(η³-allyl)Cl]₂ (70 mg, 0.19 mmol) wurde in THF (10 ml) gelöst, mit AgBF₄ (80 mg, 0.41 mmol) versetzt und eine Stunde gerührt. Die Mischung wurde filtriert, dem Filtrat das Amin zugesetzt (48 mg, 0.38 mmol) und für 30 min gerührt. Dabei fiel ein weisser Niederschlag aus. Durch weitere Zugabe von 20 ml Hexan wurde weiteres Produkt gefällt. Die Lösung wurde filtriert, mit Hexan (2 x 20 ml) und Diethylether (2 x 20 ml) gewaschen und der Rückstand im Vakuum getrocknet, wodurch ein weisses Pulver erhalten wurde (100 mg, 73 % d. Th.).
Anal: Berechnet: C₁₀H₂₁N₂PdBF₄: C, 33.14; H, 5.80; N, 7.73. Gefunden: C, 33.40; H, 5.81; N, 7.55.
¹H NMR (CD₃OD): 1.30-4.0 (m, C*H*₂ Allyl und Amin, 18H) 5.55 (m, C*H*, 1H).
¹³C NMR (CD₃OD): 14.8(1), 22.2(4), 25.3(5), 44.2(7), 50.1(2), 53.2(3), 60.3 (CH₂ allyl), 69.9(6) 116.3(CH, allyl).
ESI = 275 (M⁺), 233 (M⁺ - allyl)

### Beispiel 2

### Herstellung von kovalent immobilisiertem [Palladium(η³ -Allyl)((S)-2-Aminomethyl-1-ethylpyrrolidin)]BF₄

### a) Aktivierung von MCM-41

Zu getrocknetem, calciniertem MCM-41 (2.0 g) in THF (15 ml) wurde Dichlordiphenylsilan (0.48 g) gegeben und für eine Stunde gerührt. Die Lösung wurde dann auf -78°C abgekühlt und mit 3-Brompropyltrichlorsilan (1.10 g) versetzt. Es wurde langsam auf Raumtemperatur erwärmen gelassen und weitere 8 Stunden gerührt. Anschließend wurde eine Stunde bei 50°C gerührt. Der aktivierte Träger MCM-41 wurde abfiltriert und durch Soxhlet-Extraktion unter Verwendung von THF gereinigt. Abschließend wurde im Vakuum getrocknet.
Anal: C, 4.70; H, 0.87.

### b) Verknüpfung mit (S)-2-Aminomethyl-1-ethylpyrrolidin

Der aktivierte Träger MCM-41 (700 mg) wurde in THF (15 ml) zusammen mit dem Amin (0.15 ml) bei 50°C für 20 Stunden erwärmt. Danach wurde das Produkt filtriert und durch Soxhlet-Extraktion unter Verwendung von THF gereinigt. Abschließend wurde im Vakuum getrocknet.
Anal: C, 9.82; H, 2.0; N, 1.51.

### c) Komplexierung mit Palladium

In einem Schlenk-Gefäß wurden [PdCl(allyl)]₂ (30 mg, 0.08 mmol ) und AgBF₄ (30 mg, 0.15 mmol) in THF (10 ml) gelöst und für eine Stunde gerührt. Die Lösung wurde filtriert, mit dem modifizierten MCM-41 aus b) versetzt und vier Stunden gerührt. Der weiße Feststoff wurde filtriert, mit THF (4 x 20 ml) gewaschen und im Vakuum getrocknet.
Anal: C, 11.67; H, 2.32; N, 1.46.

### Beispiel 3 (zum Vergleich)

### Herstellung von [Palladium(η³ -Allyl)((1R,2R)-1,2-Diphenylethylendiamin) ]BF₄

[Pd(η³-allyl)Cl]₂ (70 mg, 0.19 mmol) wurde in THF (10 ml) gelöst, mit AgBF₄ (80 mg, 0.41 mmol) versetzt und eine Stunde gerührt. Die Mischung wurde filtriert, dem Filtrat das Amin zugesetzt (80 mg, 0.38 mmol) und für 30 min gerührt. Dabei fiel ein weisser Niederschlag aus. Durch weitere Zugabe von 20 ml Hexan wurde weiteres Produkt gefällt. Die Lösung wurde filtriert, mit Hexan (2 x 20 ml) und Diethylether (2 x 20 ml) gewaschen und der Rückstand im Vakuum getrocknet, wodurch ein weisses Pulver erhalten wurde (156 mg, 88 % d. Th.).
Anal: Berechnet für C₁₇H₂₁N₂RhBF₄: C, 45.74; H, 4.71; N, 6.27. Gefunden: C, 45.25; H, 4.62; N, 5.98.
¹H NMR (CD₃OD) 2.98 (m, *CH*ₐₙₜᵢ, 2H), 4.02 (s, NC*H*, 2H), 4.20 (m, C*H*_{syn}, 2H), 5.47 (m, C*H*_{central}, 1H), 7.1-7.25 (m, Ph, 10H).
¹³C NMR (CD₃OD) 56.72 (*C*H₂), 64.24 (N*C*H), 114.87 (*C*H), 127.0, 127.70, 128.25, 139.73 (Ph).
+ve ESI = 359 (M⁺).

### Beispiel 4

### Herstellung von kovalent immobilisiertem [Palladium(η³-Allyl)((1R,2R)-1,2-Diphenylethylendiamin) ]BF₄

### a) Aktivierung von MCM-41

Zu getrocknetem, calciniertem MCM-41 (2.0 g) in THF (15 ml) wurde Dichlordiphenylsilan (0.48 g) gegeben und für eine Stunde gerührt. Die Lösung wurde dann auf -78°C abgekühlt und mit 3-Brompropyltrichlorsilane (1.10 g) versetzt. Es wurde langsam auf Raumtemperatur erwärmen gelassen und weitere 8 Stunden gerührt. Anschließend wurde eine Stunde bei 50°C eine Stunde lang gerührt. Der aktivierte Träger MCM-41 wurde gefiltert und durch Soxhlet-Extraktion unter Verwendung von THF gereinigt. Abschließend wurde im Vakuum getrocknet.
Anal: C, 4.70; H, 0.87.

### b) Verknüpfung mit (1R,2R)-1,2-Diphenylethylendiamin

Der aktivierte Träger MCM-41 (500 mg) wurde in THF (15 ml) zusammen mit dem Amin (150 mg) für 20 Stunden unter Rückfluß erhitzt. Danach wurde das Produkt filtriert und durch Soxhlet-Extraktion unter Verwendung von THF gereinigt. Abschließend wurde im Vakuum getrocknet.
Anal: C, 9.72; H, 1.92; N, 0.15.

### c) Komplexierung mit Palladium

In einem Schlenk-Gefäß wurden [PdCl(allyl)]₂ (55 mg, 0.15 mmol ) und AgBF₄ (60 mg, 0.3 mmol) in THF (10 ml) gelöst und für eine Stunde gerührt. Die Lösung wurde filtriert, mit dem modifizierten MCM-41 aus b) versetzt und vier Stunden gerührt. Der Feststoff wurde filtriert, mit THF (4 x 20 ml) gewaschen und im Vakuum getrocknet.
Anal: C, 10.67; H, 2.31; N, 0.13.

### Beispiel 5 (zum Vergleich)

### Herstellung von [Rh(cod)((1R,2R)-1,2-Diphenylethylendiamin) ]BF₄

[RhCl(cod)]₂ (64 mg, 0.13 mmol) wurde in THF (10 ml) gelöst, AgCF₃SO₃ (50 mg, 0.26 mmol) zugegeben und die Lösung für eine Stunde gerührt. Die Lösung wurde anschließend filtriert, das Filtrat mit (1R, 2R)-1,2-Diphenylethylendiamin (50 mg, 0.26 mmol) versetzt und die resultierende Lösung eine Stunde gerührt. Anschließend wurde die Lösung im Vakuum eingeengt und mit Hexan (25 ml) versetzt, wobei das Produkt ausfiel. Es wurde filtriert, das Produkt mit Hexan (2 x 20 ml) und Diethylether (2 x 20 ml) gewaschen und im Vakuum getrocknet. Es wurde ein gelbes Pulver erhalten ( 185 mg, 90 %).
Anal: Berechnet für C₂₂H₂₈N₂RhBF₄ C, 51.76; H, 5.50; N, 5.50. Gefunden: C, 51.44; H, 5.57; H, 5.29.
¹H NMR (CD₃OD) 1.95 (br m, C*H*₂ 4H), 2.46 (br m, C*H*₂, 4H), 4.01 (s, NC*H*, 2H), 4.24 (m, C*H*, 2H), 4.35 (m, C*H*, 2H), 7.1-7.3 (m, Ph, 10H).
¹³C NMR (CD₃OD) 31.6 (*C*H₂), 66.3 (N*C*H) 81.4 (*C*H), 128.5, 129.2, 129.6, 140.5 (Ph).
+ve ESI = 423 (M⁺).

### Beispiel 6

### Herstellung von kovalent immobilisiertem [Rh(cod)((1R,2R)-1,2-Diphenylethylen-diamin)]BF₄

### a) Aktivierung von MCM-41

Zu getrocknetem, calciniertem MCM-41 (2.0 g) in THF (15 ml) wurde Dichlordiphenylsilan (0.48 g) gegeben und für eine Stunde gerührt. Die Lösung wurde dann auf -78°C abgekühlt und mit 3-Brompropyltrichlorsilane (1.10 g) versetzt. Es wurde langsam auf Raumtemperatur erwärmen gelassen und weitere 8 Stunden gerührt. Anschliessend wurde bei 50°C eine Stunde lang gerührt. Der aktivierte Träger MCM-41 wurde gefiltert und durch Soxhlet-Extraktion unter Verwendung von THF gereinigt. Abschließend wurde im Vakuum getrocknet.
Anal: C, 4.70; H, 0.87.

### b) Verknüpfung mit (1R,2R)-1,2-Diphenylethylendiamin

Der aktivierte Träger MCM-41 (500 mg) wurde in THF (15 ml) zusammen mit dem Amin (150 mg) für 24 Stunden unter Rückfluß erhitzt. Danach wurde das Produkt filtriert und durch Soxhlet-Extraktion unter Verwendung von THF gereinigt. Abschließend wurde im Vakuum getrocknet.
Anal: C, 9.72; H, 1.92; N, 0.15; Br, 1.30.

### c) Komplexierung

In einem Schlenk-Gefäß wurden [RhCI(cod)]₂ (30 mg, 0.06 mmol) und AgBF₄ (30 mg, 0.15 mmol) in THF (10 ml) gelöst und für eine Stunde gerührt. Die Lösung wurde filtriert, mit dem modifizierten MCM-41 aus b) versetzt und vier Stunden gerührt. Dabei entfärbte sich die Lösung langsam. Der gelbe Feststoff wurde filtriert, mit THF (4 x 20 ml) gewaschen und im Vakuum getrocknet.
Anal: C, 11.23; H, 2.02; N, 0.12.

### Beispiel 7 (zum Vergleich)

### Herstellung von [Rh(cod)((S)-2-Aminomethyl-1-ethylpyrrolidin]BF₄

[RhCl(cod)]₂ (100 mg, 0.20 mmol) wurde in THF (10 ml) gelöst, AgCF₃SO₃ (80 mg, 0.41 mmol) zugegeben und die Lösung für eine Stunde gerührt. Die Lösung wurde anschließend filtriert, das Filtrat mit (S)-2-Aminomethyl-1-ethylpyrrolidin (51.2 mg, 0.40 mmol) versetzt und die resultierende Lösung eine Stunde gerührt. Anschliessend wurde die Lösung im Vakuum eingeengt und mit Hexan (25 ml) versetzt, wobei das Produkt ausfiel. Es wurde filtriert, das Produkt mit Hexan (2 x 20 ml) und Diethylether (2 x 20 ml) gewaschen und im Vakuum getrocknet. Es wurde ein gelbes Pulver erhalten ( 185 mg, 90 %).
Anal.: Berechnet für C₁₅H₂₈N₂RhBF₄: C, 42.25; H, 6.57; N, 6.57. Gefunden: C, 42.79; H, 6.61; N, 6.59.
¹H NMR (CDCl₃) 1.76 (br m, 4H, C*H*_{*2*} Olefin), 2.45 (br m, 4H, C*H*_{*2*} Olefin), 1.55-3.30 (m, 14H, Amin).
¹³C NMR (CDCl₃) = 12.3 (1), 21.7 (4), 24.3 (5), 45.9 (7), 51.0 (2), 56.6 (3), 70.0 (6), 30.4, 30.7 (*C*H₂) 79.7, 83.6 (*C*H).
+ve ESI = 339 (M⁺), 231 (M⁺ - COD).

### Beispiel 8

### Herstellung von kovalent immobilisiertem [Rh(cod)((S)-2-Aminomethyl-1-ethylpyrrolidin]BF₄

### a) Aktivierung von MCM-41

Zu getrocknetem, calciniertem MCM-41 (2.0 g) in THF (15 ml) wurde Dichlordiphenylsilan (0.48 g) gegeben und für eine Stunde gerührt. Die Lösung wurde dann auf -78°C abgekühlt und mit 3-Brompropyltrichlorsilane (1.10 g) versetzt. Es wurde langsam auf Raumtemperatur erwärmen gelassen und weitere 8 Stunden gerührt. Anschließend wurde eine Stunde bei 50°C eine Stunde lang gerührt. Der aktivierte Träger MCM-41 wurde gefiltert und durch Soxhlet-Extraktion unter Verwendung von THF gereinigt. Abschließend wurde im Vakuum getrocknet.
Anal: C, 4.70; H, 0.87.

### b) Verknüpfung mit (S)-2-Aminomethyl-1-ethylpyrrolidin

Der aktivierte Träger MCM-41 (700 mg) wurde in THF (15 ml) zusammen mit dem Amin (0.15 ml) bei 50°C für 20 Stunden erwärmt. Danach wurde das Produkt filtriert und durch Soxhlet-Extraktion unter Verwendung von THF gereinigt. Abschließend wurde im Vakuum getrocknet.
Anal: C, 9.82; H, 2.0; N, 1.51.

### c) Komplexierung mit Rhodium

In einem Schlenk-Gefäß wurden [RhCI(cod)]₂ (30 mg, 0.06 mmol) und AgBF₄ (30 mg, 0.15 mmol) in THF (10 ml) gelöst und für eine Stunde gerührt. Die Lösung wurde filtriert, mit dem modifizierten MCM-41 aus b) versetzt und vier Stunden gerührt. Der weiße Feststoff wurde filtriert, mit THF (4 x 20 ml) gewaschen und im Vakuum getrocknet.
Anal: C, 12.45; H, 2.45; N, 1.60.
Das ¹⁹F MAS-NMR-Spektrum bestätigte die Anwesenheit des BF₄⁻ - Anions.

### Beispiele 9 bis 15:

### Allgemeine Arbeitsvorschrift für die Verwendung der Katalysatoren in asymmetrischen Hydrierungen

Die asymmetrischen Hydrierungen wurden in einem Hochdruckautoklaven aus rostfreiem Edelstahl mit einem Volumen von 150 ml durchgeführt. Jeweils 10 mg des homogenen Katalysators bzw. jeweils 50 mg der immobilisierten Katalysatoren wurden unter Inertatmosphäre in den Hochdruckautoklaven transferiert.
Phenylglyoxylsäuremethylester (0.5 g), Methanol (30 g), und ein interner Standard (Cyclododecan) wurden zugegeben und der Hochdruckautoklav verschlossen. Der Hochdruckautoklav und seine Zu- und Ableitungen wurden anschließend durch dreimaliges Spülen mit Stickstoff inertisiert und zur Prüfung der Dichtigkeit schließlich unter einen Wasserstoffdruck von 5 bar gesetzt. Anschliessend wurde der Wasserstoffdruck auf 20 bar erhöht, der Hochdruckautoklav auf Reaktionstemperatur gebracht (313 K) und der Inhalt mit einem mechanischen Rührer bei 400 U/min gerührt.
Über ein miniaturisiertes automatisches Entnahmeventil wurden Proben des Inhalts entnommen um den Verlauf der Reaktion untersuchen zu können. Am Ende der Reaktion wurde der Hochdruckautoklav zwei Stunden im Eisbad gekühlt, entspannt und die Produkte durch Gaschromatographie (GC, Varian, Model 3400 CX) über eine chirale Säule identifiziert (Chiraldex, 20 m x 0.25 mm).

Die Ergebnisse der Hydrierungsexperimente sind in nachstehender Tabelle zusammengefasst:

| **Beispiel** | **Katalysator aus Beispiel** | **Temperatur [°C]** | **Reaktionsdauer [h]** | **Umsatz [%]** | **ee [%]** |
|---|---|---|---|---|---|
| 9 | 1 (z. Vgl.) | 40 | 2 | 97,3 | 0 |
| 10 | 1 (z. Vgl.) | 40 | 24 | 99,0 | 0 |
| 11 | 2 | 40 | 2 | 99,1 | 94,5 |
| 12 | 5 (z. Vgl.) | 40 | 2 | 82,0 | 0 |
| 13 | 7 (z. Vgl.) | 40 | 2 | 94,0 | 0 |
| 14 | 8 | 40 | 2 | 97,7 | 77,5 |
| 15 | 8 | 40 | 24 | 98,6 | 80,4 |

## Patentansprüche

1. Verbindungen der Formel (I), 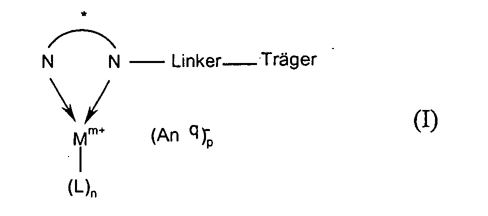 in der 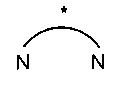 für eine enantiomerenangereicherte chirale Stickstoffverbindung steht,
Linker für einen Rest steht, der sowohl kovalent an die enantiomerenangereicherte chirale Stickstoffverbindung als auch an den Träger gebunden ist,
Träger für ein mikro-, meso- oder makroporöses Trägermaterial steht,
(M ^{m+}) für ein Metall mit der Wertigkeit m steht
L für einen anionischen oder neutralen Liganden steht
n für eins, zwei, drei oder vier steht
(An ^{q-}) für ein Anion mit der Wertigkeit q steht und
p (m - Anzahl der anionischen Liganden L)/q ist.

2. Verbindungen nach Anspruch 1, **dadurch gekennzeichnet, dass** 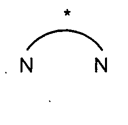 für eine enantiomerenangereicherte chirale Stickstoffverbindung der Formel (II) steht 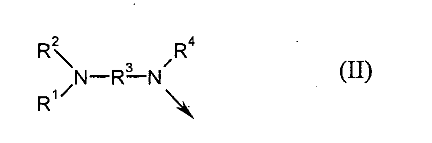 in der
der Pfeil die Bindungsstelle zum Linker anzeigt und
R¹, R² und R⁴ jeweils unabhängig voneinander für Wasserstoff, C₁-C₈-Alkyl, C₅-C₁₅-Arylalkyl oder C₄-C₁₄-Aryl stehen oder NR¹R² als Ganzes für einen cyclischen Aminorest mit insgesamt 4 bis 20 Kohlenstoffatomen steht,
R³ für einen divalenten Rest mit 2 bis 30 Kohlenstoffatomen steht oder
R³ und mindestens einer der Reste R¹, R² und R⁴ zusammen Teil eines cyclischen Aminorestes mit insgesamt 4 bis 20 Kohlenstoffatomen sind.

3. Verbindungen nach Anspruch 2, **dadurch gekennzeichnet, dass** R¹, R² und R⁴ jeweils unabhängig voneinander für Wasserstoff, C₁-C₈-Alkyl, C₅-C₁₅-Arylalkyl oder C₄-C₁₄-Aryl stehen oder NR¹R² als Ganzes für einen 5- oder 6-gliedrigen monocyclischen Aminorest steht, der am Kohlenstoffgerüst gegebenenfalls einfach, zweifach, dreifach oder vierfach C₁-C₄-Alkyl substituiert ist und
R³ für einen divalenten Rest steht, der ausgewählt ist aus der Gruppe C₂-C₈-Alkylen, das gegebenenfalls einfach oder zweifach durch C₄-C₁₄-Arylreste weiter substituiert sein kann, C₅-C₁₅-Arylalkylen, C₄-C₁₄-Arylen oder Bis-(C₄-C₁₄-arylen) oder
R³ und einer der Reste R¹, R² und R⁴ zusammen Teil eines 5- oder 6-gliedrigen monocyclischen Aminorestes sind, der am Kohlenstoffgerüst gegebenenfalls zusätzlich einfach, zweifach, dreifach oder vierfach durch C₁-C₄-Alkyl substituiert ist.

4. Verbindungen nach mindestens einem der Ansprüche 1 bis 3, **dadurch gekennzeichnet, dass** Träger für ein mikro- oder mesoporöses Trägermaterial steht.

5. Verbindungen nach mindestens einem der Ansprüche 1 bis 4, **dadurch gekennzeichnet, dass** Träger Silica-Gele oder Zeolithe vom Typ MOR, X, Y, MCM, ZSM, FAU, MFI, L, BEA, FER, A und SBA oder solche vom Typ AlPO, MA1PO und SAPO sind, wobei die genannten Zeolithe gegebenenfalls isomorph substituiert sein können.

6. Verbindungen nach mindestens einem der Ansprüche 1 bis 5, **dadurch gekennzeichnet, dass** Träger mesoporöse Zeolithe sind.

7. Verbindungen nach mindestens einem der Ansprüche 1 bis 6, **dadurch gekennzeichnet, dass** (M ^{m+}) für Cobalt in den formalen Oxidationsstufen 0, +2 und +3, Rhodium und Iridium in den formalen Oxidationsstufen +1 und +3, Nickel, Palladium und Platin in den formalen Oxidationsstufen 0 und +2 sowie Ruthenium in der formalen Oxidationsstufe +2 stehen kann.

8. Verbindungen nach mindestens einem der Ansprüche 1 bis 7, **dadurch gekennzeichnet, dass** L für folgende Ligandentypen steht: Monoolefine, Diolefine, Nitrile, Aromaten sowie anionische Liganden.

9. Verbindungen nach mindestens einem der Ansprüche 1 bis 8, **dadurch gekennzeichnet, dass** (An ^{q-}) für Nitrat, Perchlorat, Sulfat, Hexafluorophosphat, Hexafluoroantimonat, Hexachloroantimonat, Borate oder Sulfonate steht.

10. Verbindungen nach mindestens einem der Ansprüche 1 bis 9, **dadurch gekennzeichnet, dass** sie den Formeln (Ia), (Ib), (Ic) und (Id) gehorchen 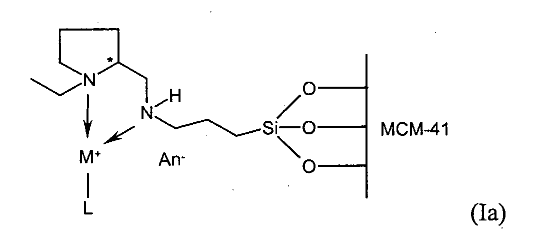 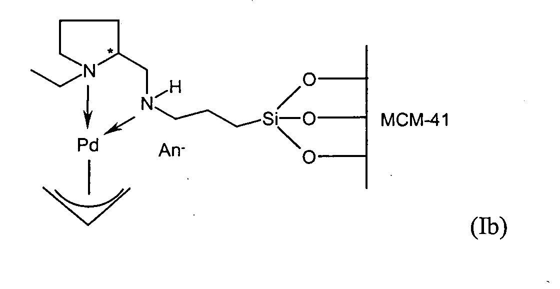 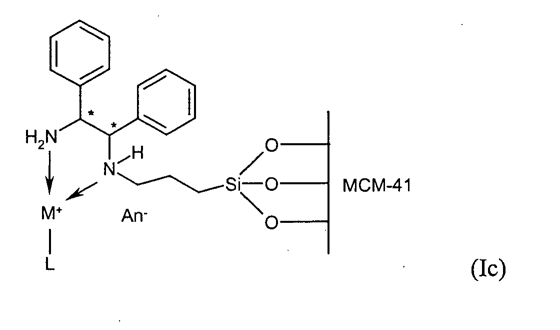 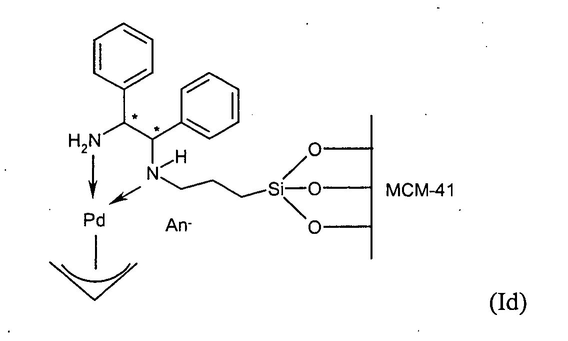 in denen jeweils
* ein stereogenes Zentrum markiert, das entweder R oder S konfiguriert ist, wobei die Auflage gilt, dass Mesoformen ausgeschlossen sind (Verbindungen der Formel (Ic) und (Id))
M⁺ für Rhodium^{I} oder Iridium^{I} steht und
L für cod oder nbd steht und
An⁻ für Perchlorat, Hexafluorophosphat, Trifluormethansulfonat oder Tetrafluoroborat steht.

11. Verbindungen der Formel (V) 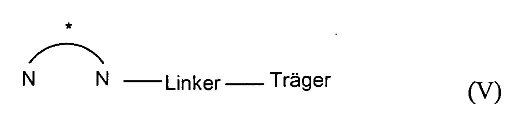 in der 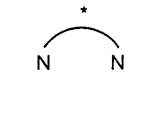 für eine enantiomerenangereicherte chirale Stickstoffverbindung steht,
Linker für einen Rest steht, der sowohl kovalent an die enantiomerenangereicherte chirale Stickstoffverbindung als auch an den Träger gebunden ist,
Träger für ein mikro-, meso- oder makroporöses Trägermaterial steht, das durch den Linker modifiziert ist.

12. Katalysatoren, enthaltend Verbindungen gemäß einem oder mehreren der Ansprüche 1 bis 10.

13. Verwendung von Verbindungen gemäß einem oder mehreren der Ansprüche 1 bis 10 als Katalysator für asymmetrische Reaktionen.

14. Verfahren zur katalytischen Herstellung von enantiomerenangereicherten Verbindungen, **dadurch gekennzeichnet ist, dass** als Katalysatoren solche eingesetzt werden, die Verbindungen gemäß einem oder mehreren der Ansprüche 1 bis 10 enthalten.

15. Verfahren nach Anspruch 14, **dadurch gekennzeichnet, dass** Verfahren zur Herstellung von enantiomerenangereicherten Verbindungen asymmetrische Hydrierungen sind.

16. Verfahren nach Anspruch 14, **dadurch gekennzeichnet, dass** asymmetrische Hydrierungen, Hydrierungen von α- und β-Ketocarbonsäureestern sind.

17. Verfahren nach Anspruch 16, **dadurch gekennzeichnet, dass** α- und β-Ketocarbonsäureester solche der Formel (VII) sind, 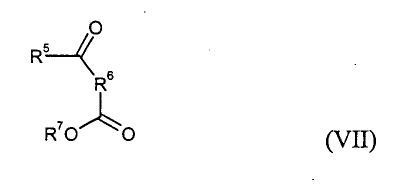 in der
R⁵ und R⁷ jeweils unabhängig voneinander für C₁-C₁₂-Alkyl, C₁-C₁₂-Halogenalkyl, C₅-C₁₅-Arylalkyl oder C₄-C₁₄-Aryl stehen und
R⁶ fehlt oder für 1,1-(C₁-C₄-Alkylen) steht.

18. Verfahren nach mindestens einem der Ansprüche 15 bis 17, **dadurch gekennzeichnet, dass** die Reaktionstemperatur bei asymmetrischen Hydrierungen 0 bis 200°C und der Wasserstoffpartialdruck 0,1 bis 200 bar beträgt.

19. Verfahren nach mindestens einem der Ansprüche 15 bis 18, **dadurch gekennzeichnet, dass** als Lösungsmittel aliphatische oder aromatische, gegebenenfalls halogenierte Kohlenwasserstoffe, Ether und/oder Alkohole eingesetzt werden.

20. Verfahren nach mindestens einem der Ansprüche 14 bis 19, **dadurch gekennzeichnet, dass** das Gewichtsverhältnis von Verbindungen gemäß mindestens einem der Ansprüche 1 bis 10 zu Substrat 1:1 bis 1:10000 beträgt.

21. Verwendung von Verbindungen, die nach einem Verfahren gemäß mindestens einem der Ansprüche 14 bis 20 hergestellt wurden als Racematspaltungsreagenzien oder in einem Verfahren zur Herstellung von Arzneimitteln oder Agrochemikalien.
